# EUROPEAN PATENT SPECIFICATION

(11) **EP 2 501 821 B1**
(45) Date of publication and mention of the grant of the patent: **15.06.2016**
(21) Application number: 10795761.5
(22) Date of filing: 29.10.2010
(51) Int. Cl.: C12P 19/62

(54) **PROCESS FOR PRODUCING PRIMYCIN, PRIMYCIN COMPONENT(S), PRECURSORS AND METABOLITES THEREOF VIA FEMENTATION BY THE USE OF BACTERIAL SPECIES SACCHAROMONOSPORA AZUREA**
VERFAHREN ZUR HERSTELLUNG VON PRIMYCIN, DESSEN VORLÄUFERN UND/ODER METABOLITEN DURCH FERMENTATION UNTER VERWENDUNG VON SACCHAROMONOSPORA AZUREA
PROCÉDÉ POUR PRODUIRE DE LA PRIMYCINE, UN OU DES COMPOSANTS DE PRIMYCINE, DES PRÉCURSEURS ET DES MÉTABOLITES DE CELLE-CI PAR FERMENTATION AU MOYEN D'ESPÈCES BACTÉRIENNES SACCHAROMONOSPORA AZUREA

(30) Priority: 29.10.2009 HU 0900678
(43) Date of publication of application: 26.09.2012
(73) Proprietor: Pannonpharma Gyógyszergyártó Kft., 7720 Pécsvárad (HU)
(72) Inventor: JUHÁSZ, Ákos, H-6000 Kecskemét (HU); PÉNZES, Ágota, H-9552 Vásárosmiske (HU); PÉTERI, Zsanett, Adrienn, H-2243 Kóka (HU); PALLOS, József, Péter, H-1221 Budapest (HU); SEFFER, Dénes, H-7720 Pécsvárad (HU); FEISZT, Péter, H-7626 Pécs (HU); PESTI, Miklós, Péter, H-7624 Pécs (HU); FEKETE, Csaba, H-7624 Pécs (HU); VÁGVÖLGYI, Csaba, H-6771 Szöreg (HU); GAZDAG, Zoltán, H-7632 Pécs (HU); PAPP, Gábor, H-7200 Dombóvár (HU)
(74) Representative: Molnar, Istvan
(86) International application number: PCT/HU2010/000116
(87) International publication number: WO 2011/051741

(56) References cited:
- US-A- 3 498 884
- CHO M ET AL: "Application of the ribonuclease P (RNAse P) RNA gene sequence for phylogenetic analysis of the genus saccharomonospora", INTERNATIONAL JOURNAL OF SYSTEMATIC BACTERIOLOGY, SOCIETY FOR GENERAL MICROBIOLOGY, READING, GB, vol. 48, 1 January 1998 (1998-01-01), pages 1223-1230, XP002955970, ISSN: 0020-7713

## Description

### Brief description of the invention

The present invention relates to a process for producing primycin, primycin producing strain(s), primycin component(s), precursors or metabolites thereof by fermentation, and application of the *Saccharomonospora azurea* to produce primycin or its component(s).

### State of art

Primycin, first described by Vályi-Nagy et al. in 1954 in Nature, was not identical with any known antibiotic according to its chemical and biological properties. [Vályi-Nagy, T., Uri, J., Szilágyi, I. (1954): Nature; 174, 1105.] The primycin producing strain was isolated from the urinary tract of the Wax Moth (*Galleria mellonella*), and on the basis of morphological properties was classified as a member of the genus *Actinomicetes*. The authors described the optimal growth, and antibiotic production conditions (media composition, temperature, pH) of the strain, they also gave the short description of the isolation and purification steps. According to the patent published in 1961 primycin was produced by fermentation of *Streptomyces* primycin "yeast strain" submerged culture in liquid medium containing nitrogen, carbon sources and inorganic salts. At the end of the process a brownish yellow, characteristic odour, alkaline fermentation broth was yielded. The isolation of the active ingredient from the fermentation broth was done by either direct extraction with water immiscible alcohols, or by so-called precipitating adsorption.

It was also determined that the active ingredient according to formula C₁₉H₃₇O₇N is heat stable, poorly soluble in water, has better solubility profile in organic solvents, and has bacteriostatic effect against Gram-positive bacteria and *Mycobacterium* species, while it is not shown to be toxic in *in vitro* cell cultures.

The first steps of the description and identification of the producing strain were taken by Vályi-Nagy *et al*.[Vályi-Nagy, T., Uri, J., Szilágyi, I. (1956): Pharmazie; 11, 304.].
Since primycin producing capacity of the formerly isolated strain in nitrogen rich media was low (1-25 µg/ml) [Vályi-Nagy, T., and Kulcsár, G. (1669): Acta biol. Acad. Sci. hung. 20 (2) 127-140.] and the strain improvement remained unsuccessful, Vályi-Nagy *et al.* isolated a new primycin producing strain and established that the new strain is not identical with the previously described *Streptomyces primycini* according to its micromorphological characteristic. The aerial mycelium of this strain did not possess the characteristics of the strain *Streptomyces* (fragmented mycelium), but the round or oval spores developed on the short ramifications of the mycelium. The newly isolated strain surpasses all varieties of S. *primycini* with its antibiotic-production yield (250-300 µg/ml). The authors established that the colour of new strain's colonies may alter from white up to dark green according to the nutrient medium and mycelium age. The colonies are round-shaped, lentil-sized, protruding and spheroidal, but due to the spontaneous mutation concentrically ribbed or irregularly wrinkled colonies may also appear. It is known from the patent description based on the newly isolated strain that primycin can economically be obtained at industrial scale (2000-3000 µg/ml) by the mutagenesis and selection of the wild type strain. The patent specification states that, regardless of the shape, size and colour of the colonies the tested all colour variants (white, green and yellow colonies) in the adequate nutrient media showed good fermentation results. The fermentation was done at 28 °C but it is noted that at higher temperature (37 °C) faster growth and higher yield should be reached. [Vályi-Nagy, T., Kulcsár, G., Szilágyi, I., Valu, G., Magyar, K., Horváth, I., Hegyaljai-Kiss, G. (1966); 153593. patent number Hungarian patent #153593].

Blaskó K. et al. [Gen Physiol Biophys. 1986 Dec; 5 (6): 625-36.] presented red blood cell membranes treated with gramicidin, primycin and valinomycin and compared it with that obtained as artificial lipid bilayer membranes. The channel forming antibiotics gramicidin and primycin show specific kinetic behaviour in living cell membranes. Based on the results it can be shown that the penetration of these antibiotics into the red blood cell membrane is a cooperative process resulting in the occurrence of aggregates in the lipid lattice of the membrane.

Uri J. V. [Acta Microbiol Hung. 1986; 33 (2):141-6.] experimentally recognized that crystalline primycin is very active. In broth dilution assay against *Staphylococcus aureus* (50 strains), *Staphylococcus epidermidis* (77 strains), *Streptococcus faecalis* (76 strains) and one strain of *Listeria monocytogenes* with minimal inhibitory concentrations (MIC) of 0.12-0.5 mg/L. The activity was influenced by the pH of the liquid medium with higher activity (lower MICs) at pH8 against the majority of strains, than at pH=6 or 7.3. In disc agar diffusion assay *Bacillus subtilis* ATCC 6633 proved more sensitive than *Staphydococcus* strains.

Sugár IP. et al. [Membrane Biochemistry. 1989; 8 (1): 1-10.] compared the erythrocytes' membrane permeability using primycin and gramidicin antibiotics. The researchers found that both antibiotics cause a non linear effect on the erythrocyte membrane permeability. The above-mentioned antibiotic treshold concentration, which is determined by the antibiotic type, increases sharply the cation permeability of the erythrocyte membrane. The non-linear transport-kinetic curves are equalized before they reach the stretch equilibrium of the radioactive ion transport between the extra-and intracellular matrix. According to a stochastic model the antibiotic molecules pass into the membrane as a result of their cooperative and aspecific co-operation which explains the results of the experiment. The authors established that the membrane permeability increases on that locations where two or more antibiotic molecules accumulate in the membrane.

Papp et. al. [Therapia Hungarica (English edition) 1990; 38 (3): 125-8] has treated superficial burns infected by Gram-positive bacteria with Ebrimycin gel containing primycin-sulphate using gauze bandage. According to the observations of 50 hospitalized and outpatients, the Ebrimycin gel has been successfully used for the local treatment of superficial bums.

P8504526 Hungarian patent application discloses a process for the production of a primycin-containing colloidal base gel. In brief, 5-30 weight% of primycin consisting of the components according to formula (I) or Formula drawing 1 wherein R1 and R2 as disclosed in the present specification was dissolved in 95-70 weight% N-methyl-pyrrolidone at a temperature of 50-150°C, and if desired, was stirred and cooled to room temperature.

Hungarian patent 158241 relates to a process for producing an antibiotic composition prepared by combining primycin and at least one another antibiotic, which is itself less effective and/or has different effectiveness against different microorganisms. This invention exploits quantitative and qualitative synergism between different antibiotics.

Hungarian patent application P8201870 relates to a process for the separation and optionally purification of primycin and mineral acid salts thereof, via extraction of the mycelium separated from the fermentation broth of the MNG-00181 strain, with a C₁₋₄ alkanol optionally recrystallisation thereof, wherein the extraction and optionally the recrystallisation is carried out with a C₁₋₄ alkanol containing trichloracetic acid. If desired, primycin base can be recovered from the primycin sulphate prepared in the above steps using known processes, or if desired its mineral acid salts can be formed by known methods.

WO9844914 international patent application relates to ointment composition for the treatment of bums and external ulcers. The ointment may contain as active ingredient a mixture of macrolide antibiotics (primycin-sulphate) or chloramphenicol or thiamphenicol together with zinc-oxide, camomile oil and azulene.

EP0182851 European patent relates to the process for the separation of the compounds of a primycin antibiotic complex. The antibiotic complex is effective against Gram-positive bacteria and polyresistant pathogenic strains.

Hungarian patent 205366 relates to a process for the separation of water soluble primycin complexes and their components as single components or as a mixture of two components. Furthermore, the specification relates to pharmaceutical compositions containing the above-mentioned components as active ingredient.

WO2007061529 intenational patent relates to a lyophilization method, which is suitable for the extraction of water-insoluble (like primycin-sulphate) substances.

At present there are 8 written and accepted *Saccharomonospora* genus members: *Saccahromonospora azurea* [Runmao 1987], DSM 44631, *Saccharomonospora cyanea [Runmao et al.* 1988], DSM 44106, *Saccharomonospora glauca* [Greiner-Mai *et.al.* 1988], DSM 43769, *Saccharomonospora viridis* [Nonomura and Ohara 1971], DSM 43017, *Saccharomonospora xinjiangensis* [Jin et al. 1998], DSM 44391, *Saccharomonospora halophila* [Al-Zarban *et al.* 2002], DSM 44411, *Saccharomonospora paurometabolica* [Li *et al.* 2003], DSM 44619 and *Saccharomonospora saliphila* [Syed *et al.* 2008], DSM 45087.

European patent specification EP1227158 describes a method for degrading polylactide resins. The polylactide resins are degraded by a microorganism belonging to the genus *Actinomycetes,* especially the species *Sacchanothrix, Streptoalloteichus, Kibdelosporangium, Lentzea, Actinokineospora, Saccharomonospora, Saccharopolyspora, or Actinopolyspora.* The claims also mention *Saccharomonospora azurea* species.

European patent EP104141 describes a novel process for the isolation and application of a bacteriophage from universal growth medium. The process is useful to investigate the details of biological, biochemical, physiological and genetic properties of actinomycetes and among these *Saccharomonospora* strains.

WO0059307 international patent application relates to a process for the preparation of a dough or a baked product which comprises adding amylase to the dough in order to retard the staling of the bread. The added amylase can be an amylase enzime where the initial product of the hydrolysis contains maltose and maltotriose as main products. Such an enzime can be obtained from a strain selected from *Actinomycetales*, *Streptosporangineae*, *Micromono-sporineae* or *Pseudonocardineae*, in particular *Thermomonospora*, *Micromonospora* or *Saccharomonospora,* more specifically species *Thermomonospora curvata, Thermomonospora viridis, Micromonospora melanosporea* or *Saccharomonospora. viridis*.

### Object of the invention

Our object was to discover an alternative method for the production of primycin, or primycin component(s), using an alternative primycin producing bacterium species.

In our research leading to our new findings we assayed the antimicrobial spectrum of primycin in order to re-evaluate the minimal inhibitory concentration (MIC) values known from the literature and to determine the primycin susceptibility of new isolates. Comparative test results on strains of Gram-positive bacterial species of Microbial strain culture collection of PTE TTK BI Department of General and Environmental Microbiology were in agreement with the literature data with primycin MIC values of 0.06 mg/L for *Staphylococcus sp.* and 0.03 mg/L for *Bacillus sp.* respectively. Primycin MIC values higher than 1 mg/L for any of the Gram-positive strains tested were not detected. Primycin did not prove to be effective against Gram-negative species.

Investigating further Gram-positive species from the culture collection of the Department with no literature data on primycin susceptibility we surprisingly found that the strains of species belonging to *Saccharomonospora* genus showed primycin MIC values higher than the expected (MIC=8-64 mg/L). To confirm these results we involved further *Saccharomonospora sp.* strains in the study finding emerged primycin tolerance (MIC=64 mg/L) in cases of *Saccharomonospora azurea* DSM 44631, and *Saccharomonospora azurea (caesia)* DSM 43044, DSM 43068 and DSM 43680 strains.

Considering that we did not find primycin MIC values higher than 1 mg/L for strains of Gram-positive species, decreased primycin susceptibility did not occur, the MIC value of 64 mg/L detected in case of *Saccharomonospora aurea* strains can be considered surprisingly high. As it is known that antibiotic-producing strains are less susceptible to the antibiotic produced by themselves and the primordials of the existing primycin-producing strain also was selected by primycin tolerance level to enhance their production capability, it has been suspected that lowered primycin susceptibility of *Saccharomonospora azurea* strains can be the outcome of the production of an antibiotic with a mode of action analogue to that of primycin. The literature concordantly states that primycin has a unique mode of action, therefore it could be suspected from our surprising findings that the antibiotic supposedly produced by these strains can be primycin itself. However, there is no literature on primycin production of *Saccharomonospora azurea* or any other presently accepted species of *Saccharomonospora* genera.

To prove the theory of our new findings first we examined the antimicrobial effect of extracted cells of *Saccharomonospora sp.* strains cultivated on solid media. Strains were grown for seven days and once the colonies were removed and extracted, they were tested for antimicrobial production in agar diffusion tests. We surprisingly found that the antimicrobial effects of *Saccharomonospora azurea* cell extracts were comparable to that of primycin against other strains of Gram-positive bacterial species. For this comparison we repeatedly used the strains of the culture collection of Department which had preliminary been tested for primycin susceptibility. Our work hypothesis was further supported by our experience that a butanol-ethanol-water mixture (in the ratio of 1:1:2 respectively) which is an effective solvent of primycin was suitable for the extraction of the antibiotic. Extracts of *S. cyanea* DSM 44106, *S. viridis* DSM 43017, *S. glauca* DSM 43769, *S. xinjiangensis* DSM 44391, *S. paurometabolica* DSM 44619 and *S. saliphylica* DSM 45087 cultures did not show antimicrobial effect. Thereafter we carried out shake flask fermentations with the *Saccharomonospora sp.* strains suitable for the process. Production results on shake flasks of the strains tested were congruent to their cultivations on solid media. Since the antibiotic produced by *Saccharomonospora sp.* had an antimicrobial spectrum similar to that of primycin and it also had effect on the *Enterococcus hirae* ATCC 8043 strain used for biological assay of primycin, we attempted to quantify the antibiotic production level of the *Saccharorraonospora* strains with the biological assay of primycin. Data of *Table 1.* show clear connection between the high primycin tolerance (MIC=64 mg/L) and the antibiotic production in every case.

**Table 1. Primycin tolerance and antibiotic production of Saccharomonospora isolates. *: S. azurea strains identified and registered on the name S. caesia earlier:**

| **Species (DSM no.)** | **Primycin MIC** | **Antibiotic production** |
|---|---|---|
| *S. viridis* (43017) | 0,5 mg/L | none |
| *S. caesia* * (43044) | 64 mg/L | approx. 250 E/ml |
| *S. caesia* * (43068) | 64 mg/L | approx. 145 E/ml |
| *S. caesia* * (43680) | 64 mg/L | approx. 110 E/ml |
| *S. glauca* (43769) | 8 mg/L | none |
| *S. cyanea* (44106) | 8 mg/L | none |
| *S. xinjiangensis* (44391) | 1 mg/L | none |
| *S. azurea* (44631) | 64 mg/L | approx. 40 E/ml |

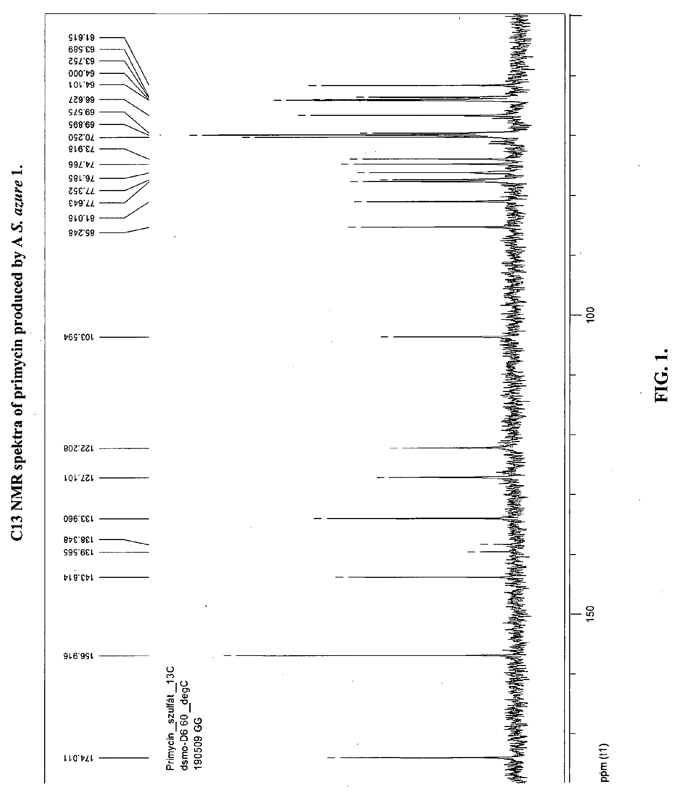

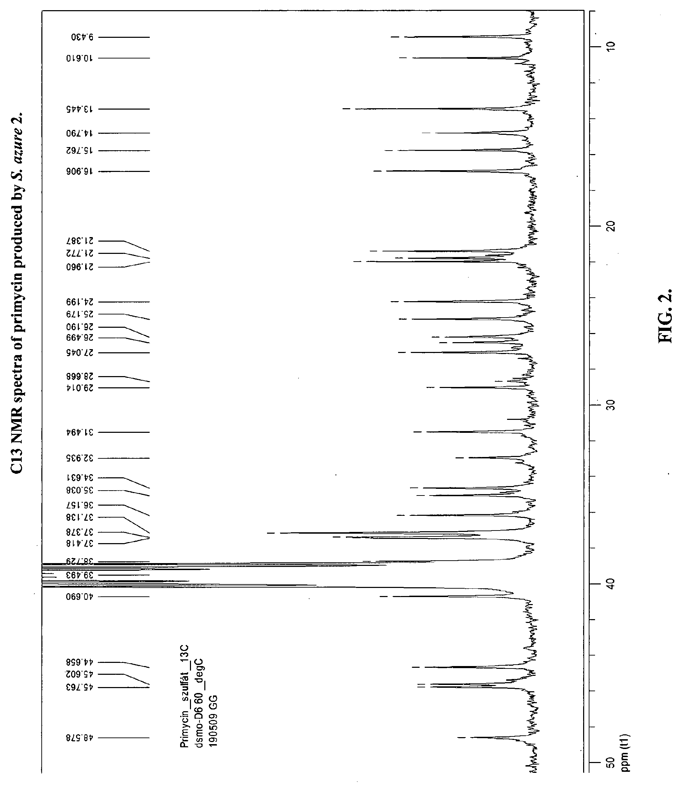

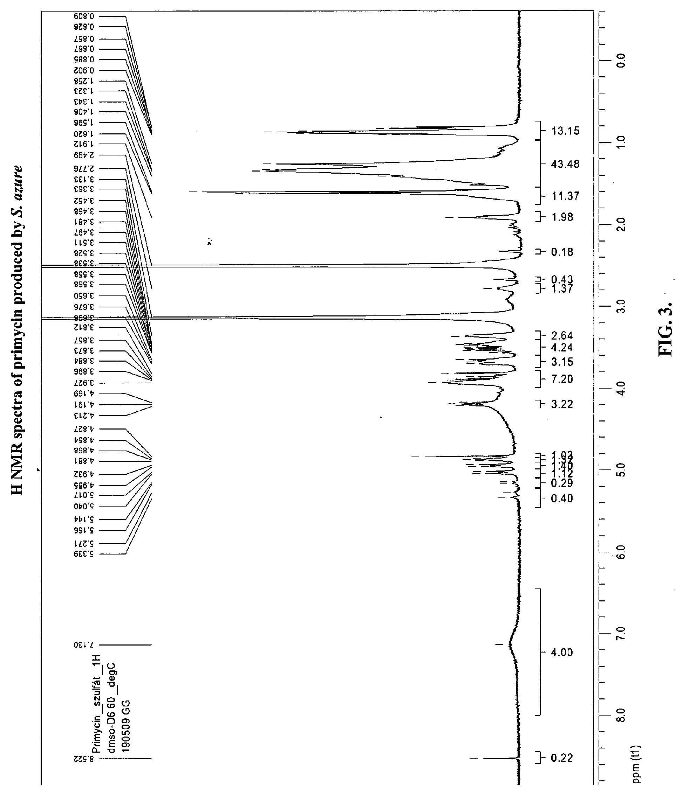

*S. azurea* (DSM 43044, 43068, 43680, 44631) strains showed prominent antibiotic producing ability. We produced antibiotic in sufficient amount for structure identification by fermentation and extraction process described in the example using the most efficiently producing *Saccharomonospora azurea (caesia)* strain. We identified the substance as primycin-sulphate based on the NMR spectra of it (*Fig. 1*.).

Consequently, the *S. azurea (caesia)* DSM 43044, 43068, 43680 and 44631 are primycin-producing strains. This information is surprising and unexpected, since the primycin production of these strains has never been published in spite of the fact that these strains has been deposited in international strain culture collections, furthermore they are well characterized commercially available strains, and the primycin antibiotic is also known since 1954.

### Brief description of the invention

The present invention relates to a process for the production of primycin, its components, wherein Saccharomonospora azurea species, any of its variants or mutants is fermented on a culturing medium, and after reaching the sufficient concentration the primycin, primycin component is recovered by known process or a combination of known process.

### Detailed description of the invention

### Definitions

The term **"primycin components",** as used in the present specification, refers to the compounds of general formula according to Formulae drawing (I), wherein R₁ is n-butyl or n-pentyl or n-hexyl, R₂ is arabinosyl or H or OH (Table 2).

**Table 2. R1 and R2 substituents of the known primycin components**

| Component | R1 | R2 |
|---|---|---|
| A1 | n-butyl | -arabinosyl |
| A2 | n-pentyl | -arabinosyl |
| A3 | n-hexyl | -arabinosyl |
| B1 | n-butyl | -H |
| B2 | n-penthyl | -H |
| B3 | n-hexyl | -H |
| C1 | n-butyl | -OH |
| C2 | n-pentyl | -OH |
| C3 | n-hexyl | -OH |

Primycin components may exist in a form of base or salt. In case of the salt form X is either an acid residue of an inorganic acid, preferably sulphate-ion, or an acid residue of an organic acid, preferably acetate-ion.

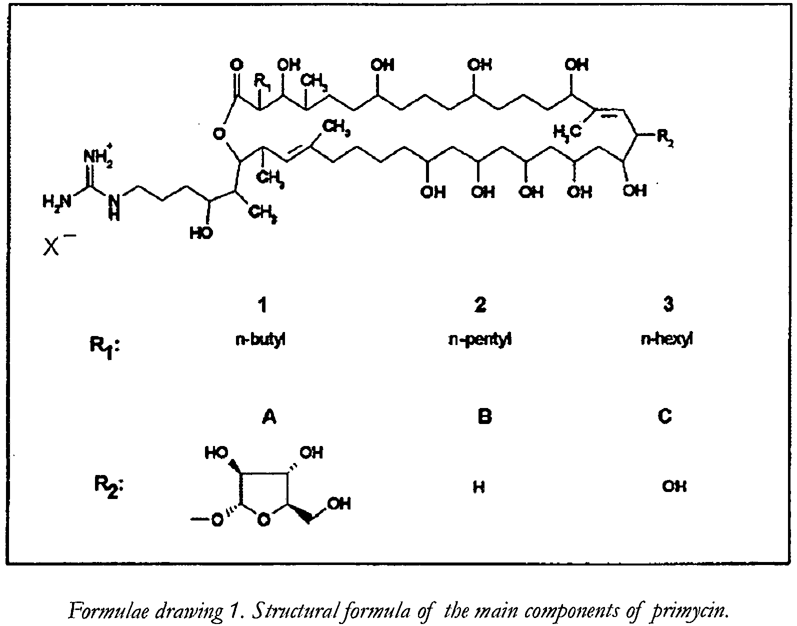

The chemical name of the sulphate-salt of primycin A1 component: 18-arabinosyl-2-butyl-3,7,11,15,19,21,23,25,27,37-dekahydroxy-4,16,32,34,36-pentamethyl-tetraconta-16,32-diene-35-O-lacton-40-guanidium-sulphate or {5-/19-(α-D-arabinofuranosyloxy)-35-butyl-10,12,14,16,18,22,26,30,34-nonahydroxy-3,5,21,33-tetramethyl-36-oxo-oxacyclohexatriaconta-4,40-diene-2-yl/-4-hydroxyhexyl}-guanidium-sulphate.
The term **"primycin"**, as used in the present specification, refers to an arbitrary proportion of at least two primycin components.

The term **"species",** as used in the present specification: two organisms belong to the same species when their gene sequences show at least 97% homology to each other investigated by DNA/DNA hybridization technique known from literature.
The meaning of term **"variation (varietas)"** is a taxonomical definition; a sub-species taxon; variations are organisms within species, which have hereditarily different genotype from wild type strain, but which did not isolate from wild strain neither geologically nor ecologically. All variation of the *Saccharomonospora azurea* belongs to the species *Saccharomonospora azurea,* taking into account the *Saccharomonospora azurea* species deponated and registered on different name.

The term **"mutant",** as used in the present specification, is a genetical definition: mutants are the totality of organisms belonging to the same species, which have different genotype from wild strain, and keep stably and transmit these properties through generations.
The term **"Saccharomonospora azurea**" (former synonym name: *S. caesia*), as used in the present specification, refers to a microorganism which belongs to *Cellular organism;*
*Superkingdom: Bacteria; Phylum: Actinobacteria; Class: Actinobacteria; Subclass: Actinobacteridae; Ordo: Actinomycetales; Subordo: Pseudonocardineae; Family: Pseudonocardiaceae; Genus: Saccharomonospora,* and which can be characterized as follows: aerobic actinomycete. Substrate mycelium nonfragmenting. Do not form sporangium. Single spores formed mainly on aerial mycelium. Spores are oval or round, 0.8 to 1.0 µm. Sporophores are very short or sessile. Spore surface is smooth. The color of the aerial mycelium is azure on oatmeal agar and Czapek Dox sucrose agar. No distinct soluble pigment is formed. Utilizes D-fructose, mannose, L-rhamnose, ribose, D-xylose, sucrose, maltose, lactose, melibiose, trehalose, D-glucose, glycerol, and raffinose, but not L-arabinose, i-inositol, D-mannitol, and galactose. Cell wall type IV. Mesophilic. Habitat: soil. Type strain: strain NA-128 (=SIA 86128) according to publication of H. Runmao (1987). We consider the cited publication authentic in respect of other characteristics.

The present invention relates, in its first aspect, to a process for the production of primycin, primycin component(s) of formula (I), wherein primycin, primycin component(s) are produced by a microorganism selected from the group consisting of *Saccharomonospora azurea*, variant strains and mutants thereof. During the process said microorganism is fermented on culture medium, and after achieving appropriate concentration, primycin, primycin component are recovered by a known process or a combination of such processes. As culture medium organic sources of carbon and nitrogen, mineral salts, natural and/or synthetic fats, oils, fatty alcohols and fatty acids (preferably stearic acid) are used. The producing strains belonging to *Saccharomonospora azurea* species are grown on the above mentioned culture medium. The fermentation is carried out at temperatures between 18° and 35°C, and at a pH value of 6 to 9, for at least 48 hours.
After achieving appropriate concentration of primycin, primycin component(s), the cells are lysed, then the product is enriched using any of the processes known from literature and/or a combination of such processes, finally recovering the product with a known process or a combination of such processes.

In a further aspect the present invention relates to the application of a microorganism selected from the group consisting of *Saccharomonospora azurea* and variant strains and mutants thereof for the production of primycin, primycin component(s).

### Examples

### Example 1: Process for producing primycin

### Laboratory scale fermentation and preparation of primycin sulphate

*Saccharomonospora azurea* strains are grown in LB culture medium at a temperature of 20-40°C, rotating with 150-300 rpm for 24-48 hours according to the optimum of the individual strain.

### Composition of LB medium:

1.0 m/m % Tryptone
0.5 m/m % Yeast extract
1.0 m/m % Sodium chloride
pH=7.5

In 500 ml Erlenmeyer flasks containing 100 ml of the culture medium, 1 ml portion of the above prepared cell culture was inoculated under sterile conditions. The flasks were then shaken at a temperature of 20-40°C with 150-300 rpm for at least 48 hours on a shaking table, according to the optimum of the individual strain.

Composition of the inoculum medium according to the optimum of the individual strain:

| | |
|---|---|
| 2.0 - 4.0 | m/m % Soya flour |
| 4.0 - 6.0 | m/m % Potato starch |
| 0.2 - 4.0 | m/m % Sodium chloride |
| 0.5 - 1.0 | m/m % Calcium carbonte |
| 0.5 | m/m % Sunflower oil |

500 ml flasks containing 100 ml of the fermentation medium were inoculated under sterile conditions with 10 ml portion of the above prepared inoculum. The flasks are than shaken at a temperature of 20-30°C with 150-300 rpm for at least 48 hours on a shaking table, according to the optimum of individual strain.

Composition of the fermentation medium according to the optimum of the individual strain:

| | |
|---|---|
| 4.0 - 6.0 m/m % | Soya flour |
| 4.0 - 6.0 m/m % | Potato starch |
| 0.2 - 4.0 m/m % | Sodium chloride |
| 0.5 - 1.0 m/m % | Calcium carbonate |
| 0.60 m/m % | Sunflower oil |
| 0.20 - 0.60 m/m % | Stearic acid |
| 0.00 - 0.20 m/m % | Potassium dihydrogen phosphate |

Preparative purification of primycin sulphate

The preparative purification is started form 500 ml fermentation broth (5 parallel fermentations).
- The fermentation broth is centrifugated (15 minutes, 5000 1/min), the supernatant is decanted.
- The above gained cell mass is extracted with 500 ml methanol containing 10 m/m% sulphuric acid in an ultrasonic water bath at a temperature of 50°C for 1 hour.
- The extract is centrifuged (15 minutes, 5000 1/min), the supernatant is decanted, evaporated to the 1/5 portion of the original volume, the residue is cooled.
- The precipitate is filtered, washed with acetone, water then cold methanol.

As a result of the above process 0.24g primycin sulphate was obtained.

### The advantage of the present invention

With our invention we have created a novel, alternative technology suitable for producing primycin, which can be carried out with application of new producing microorganisms, thereby perspectively increasing the general safeness of the primycin production.

Every available antibiotic represents potentially high social and economical value in view of the growing incidence of antibiotic resistance of the most common pathogen microorganisms and the increasing time need of the development of new antibiotics.

## Claims

1. A process for the production of primycin or primycin components, wherein said primycin components are compounds of formula (I) 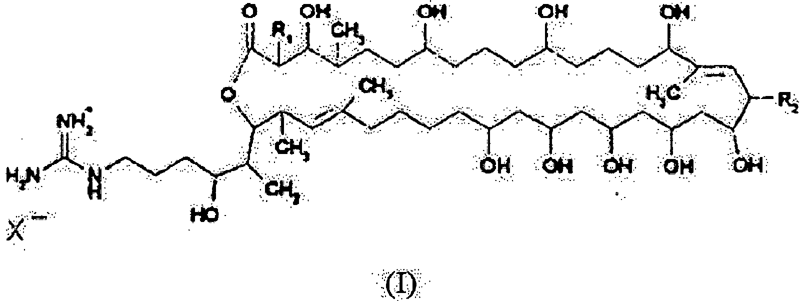 wherein
R₁ is n-butyl, n-pentyl or n-hexyl,
R₂ is H, OH or a group of formula (A) 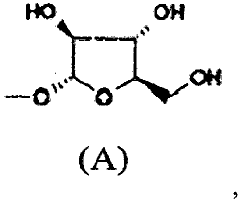 X⁻ is either an acid residue of an inorganic acid or an acid residue of an organic acid, said process comprising the steps of fermenting a microorganism selected from the group consisting of *Saccharomonospora azurea*, variant strains and mutants thereof on a culture medium, and after achieving the appropriate concentration, recovering primycin or primycin component by a known process or a combination of known processes.

2. A process according to Claim 1, wherein as culture medium a medium containing organic carbon source, organic nitrogen source, mineral salts and preferably natural or synthetic fats, oils, fatty alcohols and/or fatty acids is used.

3. A process according to Claims 1 or 2, wherein said fatty acid is stearic acid.

4. A process according to Claims 1 to 3, wherein the fermentation is carried out at a temperature of 18 to 35°C, at a pH value of 6 to 9 for at least 48 hours.

5. Use of *Saccharomonospora azurea*, variant strains and mutants thereof to produce primycin or primycin components, according to formula (1) as claimed in Claim 1.

## Patentansprüche

1. Verfahren zur Herstellung von Primycin oder Primycin-Komponenten, wobei die Primycin-Komponenten Verbindungen der Formel (I) 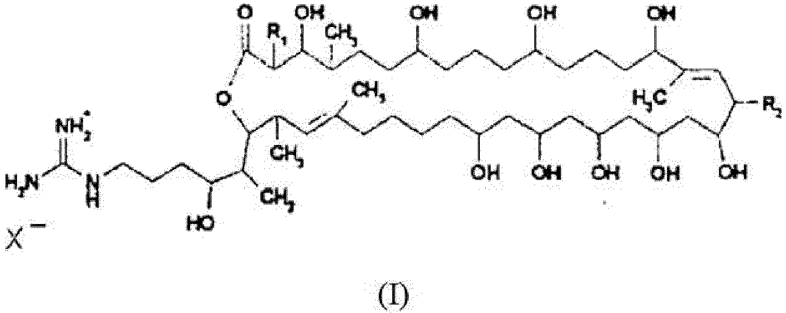 sind,
worin
R₁ für n-Butyl-, n-Pentyl oder n-Hexyl steht,
R₂ für H, OH oder Gruppe (A) 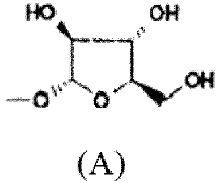
steht,
X für einen Säurerest einer anorganischen Säure oder einen Säurerest einer organischen Säure steht,
und das Verfahren die Schritte der Fermentation von Mikroorganismen ausgewählt aus der Gruppe der *Saccharomonospora azurea*, ihrer Varianten und Mutationen auf einem Nährboden, und nach dem Erreichen der entsprechenden Konzentration die Gewinnung des Primycin oder der Primycin-Komponente durch ein bekanntes Verfahren oder die Kombination von bekannten Verfahren beinhaltet.

2. Verfahren gemäß Anspruch 1, worin als Nährboden ein Nährboden verwendet wird, der eine organische Kohlenstoffquelle, organische Stickstoffquelle, Mineralsalze und vorzugsweise natürliche oder synthetische Fette, Öle, Fettalkohole und/oder Fettsäuren enthält.

3. Verfahren gemäß Anspruch 1 oder 2, worin die Fettsäure Stearinsäure ist.

4. Verfahren gemäß den Ansprüchen 1-3, worin die Fermentation bei einer Temperatur von 18-35 °C, bei einem pH-Wert von 6-9 und mindestens 48 Stunden lang erfolgt.

5. Verwendung von Saccharomonospora azurea, ihrer Varianten und Mutationen für die Herstellung von Primycin oder Primycin-Komponenten der Formel (I) gemäß Anspruch 1.

## Revendications

1. Un procédé pour la production de primycine ou de composants de primycine, où lesdits composants de primycine sont des composés de formule (I) 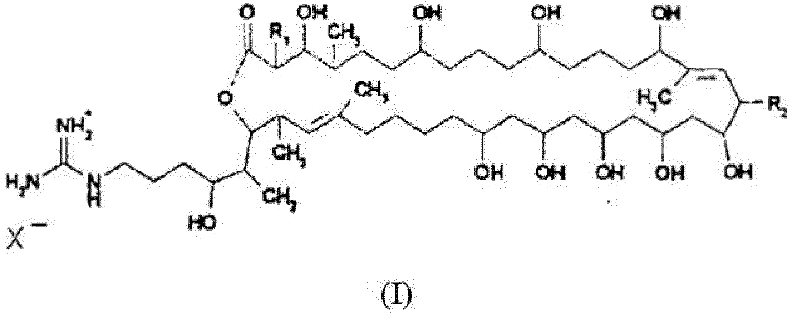 dans laquelle
R₁ est n-butyle, n-pentyle or n-héxyle,
R₂ est H, OH ou un groupe de formule (A) 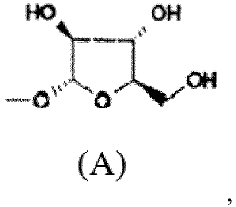 X⁻ est soit un résidu d'acide d'un acide inorganique soit un résidu d'acide d'un acide organique,
ledit procédé comprenant les étapes de fermenter un microorganisme sélectionné parmi le groupe constitué du *Saccharomonospora azurea*, des souches variantes et des mutantes de celui-ci, sur un milieu de culture, et après avoir atteint la concentration appropriée, extraire la primycine ou les composants de primycine par un procédé connu ou par une combinaison de procédés connus.

2. Un procédé selon la revendication 1, dans lequel comme milieu de culture un milieu de culture contenant des sources de carbone organique, des sources de nitrogène organique, des sels minéraux et préférablement des graisses, huiles, alcools gras et/ou acides gras naturels ou synthétiques est utilisé.

3. Un procédé selon les revendications 1ou 2, dans lequel ledit acide gras est l'acide stéarique.

4. Un procédé selon les revendications 1 à 3, dans lequel la fermentation est conduite à une température de 18 à 35°C, à une valeur de pH de 6 à 9 pendant au moins 48 heures.

5. Utilisation du *Saccharomonospora azurea*, des souches variantes et des mutantes de celui-ci, pour produire de la primycine ou des composants de primycine répondant à la formule (I) tel que revendiqués dans la revendication 1.
